# EUROPEAN PATENT SPECIFICATION

(11) **EP 0 245 304 B1**
(45) Date of publication and mention of the grant of the patent: **07.01.1993**
(21) Application number: 86906161.4
(22) Date of filing: 24.09.1986
(51) Int. Cl.: A61M 1/00

(54) **FAST RESPONSE TUBELESS VACUUM ASPIRATION COLLECTION CASSETTE**
SCHNELL ANSPRECHENDE, SCHLAUCHLOSE ASPIRATIONS-SAMMELKASSETTE
CASSETTE COLLECTRICE PAR ASPIRATION, SANS TUBES ET A REPONSE RAPIDE

(30) Priority: 25.09.1985 US 780073
(43) Date of publication of application: 19.11.1987
(73) Proprietor: ALCON LABORATORIES, INC., Fort Worth, TX 76115 (US)
(72) Inventor: SUNDBLOM, Leif, J., Castro Vally, CA 94546 (US); GEORGE, William, T., Oakland, CA 94618 (US)
(74) Representative: Kerr, Simonne June
(86) International application number: US8602006
(87) International publication number: WO8701943

(56) References cited:
- US-A- 2 211 167
- US-A- 3 019 815
- US-A- 3 613 729
- US-A- 3 850 265
- US-A- 4 064 630
- US-A- 4 144 644
- US-A- 4 223 813
- US-A- 4 236 880
- US-A- 4 324 243
- US-A- 4 475 904
- US-A- 4 479 760
- US-A- 4 493 695

## Description

### BACKGROUND OF THE INVENTION

In modern surgical practice, many procedures which formerly required large incisions have been supplanted by techniques employing microsurgical tools. Such tools are generally introduced to the surgical site through small incisions which create far less surgical trauma, and hasten healing and recovery.

A significant aspect of many of the newly developed surgical tools is the use of vacuum aspiration to draw tissue into a power driven cutting head. It is well recognized that the rate of tissue cutting and removal is related to the rate of operation of the cutting head, and also to the vacuum level applied to the aspiration port associated with the cutting head. Thus it is clear that precise control of the vacuum level is essential to successful operation of this type of surgical instrument.

Until recently it was standard practice to have tubing and hoses extending to the cutting instrument to provide aspiration, irrigation fluid, and motive power to the cutting head. The tubing generally was manually secured through pinch valves on the console of a surgical instrument control machine to provide control of the irrigation and aspiration functions. However, this procedure was subject to human error in the placement of the proper tubing in the proper pinch valve, and it was also possible for the tubing to become dislodged accidentally. The outcome of such errors can be catastrophic during surgery.

One means for overcoming this problem is disclosed in United States Patent No. 4,475,904, issued October 9, 1984 to Carl C. T. Wang. This device provided a pair of aspiration containers in a cassette housing, one container having a significantly smaller volume than the other. The containers are connected by tubing affixed to an outer wall of the cassette, and valves extending from the console of the surgical control machine are disposed to engage the tubing automatically when the cassette is secured to the machine. Also, the vacuum connections to the cassette are engaged automatically upon installation of the cassette. Thus the human error aspect is alleviated. More significantly, the assembly provides the fast response to changes in vacuum associated with a small volume while also providing a large storage capacity.

However, pinch valves mounted on the exterior of a machine exhibit intrinsic reliability problems, such as clogging by foreign matter, sticking, and the like. Furthermore, the tubing itself must be extremely resilient to expand quickly when the pinch valves open, so that the response time of the overall assembly does not lag. If a tubing section remains pinched by a valve for a long period of time, plastic deformation may occur, and the flow channel will be constricted, thus altering the operating characteristics of the surgical tool. Temperature changes may also affect the resilience of the tubing.

It is additionally problematic that many times surgery requires contemporary use of different types of surgical instruments. As previously discussed, aspiration, irrigation, and mode of power for a cutting tool require connnection to different sources. Use of tubing or hoses is susceptible to problems, as previously discussed, which is compounded by the different sources and types of tools. It would therefore be advantageous to have an integrated control center and conduit for the different sources and tools.

The invention generally comprises a vacuum aspiration collection system which features all of the conveniences and error-free aspects of a cassette system, while alleviating the problems associated with tubing and pinch valve operation. Thus the invention provides fast response to changes in demand for vacuum at the aspiration port, large capacity for storage of aspirated fluids and tissue, and highly reliable setup and operation.

According to the present invention a fast response vacuum aspiration system for use with a source of aspiration and an aspiration surgical instrument, comprises a removable cassette housing which includes a wall portion, having a first surface which, when in use, is oriented towards the source of aspiration, at least one sealed collection container secured within the cassette housing, one or more flow channels formed in the first surface of the wall portion of the cassette housing for communicating the container with the source of vacuum and the aspiration tool, and is characterized in that a resilient gasket abuts the first surface of the wall portion and covers the fluid channels, a retaining plate, associated with the cassette housing, sandwiching the gasket to the wall portion and one or more valve seats interposed along the flow channels adapted to receive a valve actuator means through the retaining plate to force a portion of the gasket into the valve seat blocking any fluid flow in the flow channels.

The invention will now be described further, by way of example, with reference to the accompanying drawings, in which
Figure 1 is a perspective view of the aspiration cassette assembly of a first preferred embodiment of the present invention.
Figure 2 is a rear view of the aspiration cassette assembly depicted in Figure 1.
Figure 3 is a side view of the aspiration cassette assembly depicted in Figures 1 and 2.
Figure 4 is a plan view of the rear panel layout of the aspiration cassette assembly of Figure 1.
Figure 5 is a cross-sectional elevation of the rear panel assembly, taken along line 5-5 of Figure 4.
Figure 6 is a cross-sectional elevation of the rear panel assembly, taken along line 6-6 of Figure 4.
Figure 7 is a cross-sectional elevation of the rear panel assembly, taken along line 7-7 of Figure 4.
Figure 8 is a cross-sectional elevation of the rear panel assembly of the cassette assembly, taken along line 8-8 of Figure 4.
Figure 9 is a partial cross-sectional elevation of the rear panel assembly of the cassette assembly, taken along line 9-9 of Figure 4.
Figure 10 is a cross-sectional view of the cassette assembly, taken along line 10-10 of Figure 8.
Figure 11 is a plan view of the valve stem and piston assembly of the present invention.
Figure 12 is a partially cross-sectioned detail view of the corner assembly of the present invention.
Figure 13 is an enlarged, cross-sectional view of the valve assembly according to the present invention of Figure 1.
Figure 14 is a partial plan view of the rear wall of the aspiration cassette of Figure 1.
Figure 15 is a cross-sectional elevation taken along line 15-15 of Figure 14.
Figure 16 is a cross-sectional elevation taken along line 16-16 of Figure 14.
Figure 17 is an enlarged, detailed cross-sectional view taken along line 17-17 of Figure 4.
Figure 18 is a detailed plan view of the valve construction of the present invention of Figure 1.
Figure 19 is a perspective view of a second preferred embodiment of the present invention.
Figure 20 is a cross-sectional elevation taken along lines 20-20 of Figure 21.
Figure 21 is a side cross-sectional elevation taken along line 21-21 of Figure 19.
Figure 22 is a top cross-sectional view taken along line 22-22 of Figure 20.
Figure 23 is a top cross-sectional view taken along line 23-23 of Figure 20.
Figure 24 is an enlarged, cross-sectional view of the vent assembly according to the present invention of Figure 19, showing venting in operation.
Figure 25 is a schematic representation of an optional auxiliary fluid container system which can be used with the present invention.

### DESCRIPTION OF THE PREFERRED EMBODIMENT

The present invention generally comprises a device for collecting aspirant fluid from a vacuum aspiration surgical instrument or probe. It can optionally also control delivery of infusion fluid to an infusion surgical instrument or probe. One salient feature of the invention is that the device includes a cassette configuration which releasably engages the surgical instrument operating machine by simple plug insertion, thereby making all the required connections with the machine. Also, the cassette includes therein all the valves necessary to control the vacuum aspiration or infusion fluid functions, thus obviating the prior art problems associated with tubing connections, pinch valves, and the like. Moreover, the invention exhibits extremely fast response time in shifting the vacuum induction level, while also providing a large storage capacity for aspirant fluid and tissue debris. Other features of the invention will be apparent in the following description.

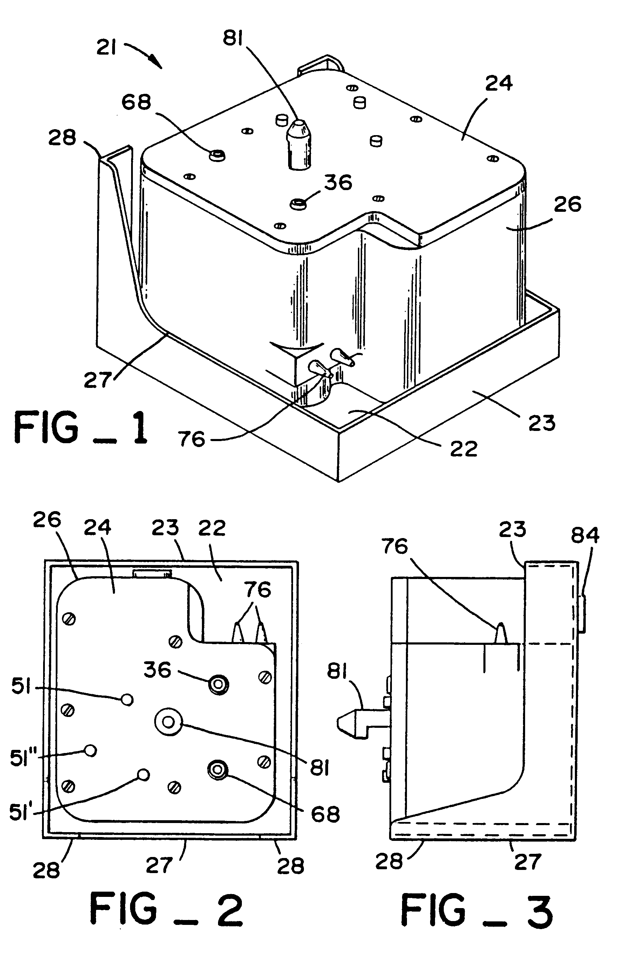

A first preferred embodiment of the invention is shown in Figures 1-18, and will be described as follows. With regard to Figures 1-3, the cassette 21 of the present invention includes an outer end wall 22 which is generally rectangular in configuration, with a narrow frame 23 extending about the periphery of the wall 22. An inner end wall 24 is parallel and spaced apart from the wall 22, and is provided with a smaller profile, slightly irregular rectangular configuration. A sidewall 26 extends continuously between the two end walls to define an enclosed volume therewith. The sidewall and end walls are preferably formed of molded polymer material, and are joined by adhesive means or ultrasonic welding to form a sealed unit. At the lower portion of the cassette, the frame 23 includes a portion 28 which flares to extend from the outer end wall to the inner end wall, adjacent to the opposed corners of the lower edge 27 of the wall 22.

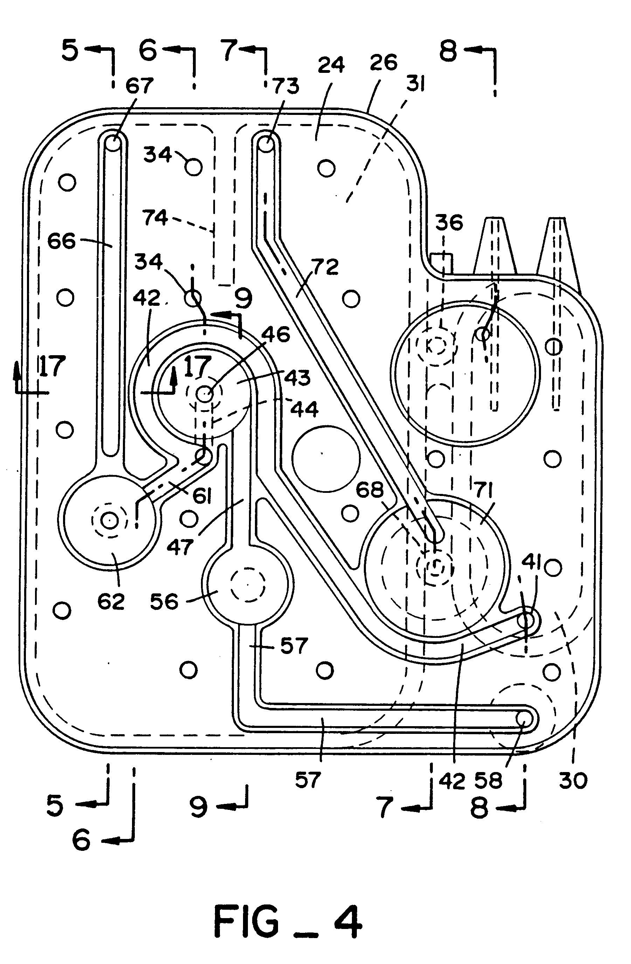

With regard to Figures 4, 8, and 10, the cassette 21 is provided with interior walls which define, together with the outer sidewalls 26, a pair of sealed containers 30 and 31. It is significant that the container 30 is small in volume, and that the container 31 is substantially larger. A system of flow channels and valves, formed by an assembly with the end wall 24, selectively connected the two containers. In addition, the smaller container 30 is connected to a controlled, variable vacuum source, and this source of vacuum is connected through the smaller container 30 to a surgical instrument or probe to aspirate fluid and tissue therefrom into the smaller container. It may be appreciated that the small volume of the container 30 closely follows the vacuum level of the source connected to it, so that the vacuum aspiration demanded by the surgeon is delivered to the surgical tool with virtually no perceptible time lag. Also, the system connects the larger container 31 to a fixed vacuum source and selectively to the smaller container, so that the smaller container may be emptied whenever it becomes full, or whenever the operation of the surgical instrument is halted even momentarily.

Joined to the end wall 24 is an inner panel 32 disposed parallel thereto and having the same general outer profile. A gasket member 33 is also provided with the same general outer profile, and is secured compressively between the wall 24 and the panel 32. The gasket 33 is formed of a resilient, inert material, such as silicone rubber or the like. A plurality of locating pins 34 extend from the inner panel 32 through appropriately placed holes in the gasket to be received in friction fit in apertures in the end wall 24. The pins 34 facilitate assembly of the device in a precision manner, and the gasket provides a pressure-tight vacuum seal. In addition, the inner panel 32 includes flow channels and valve chambers formed integrally therein during the molding process, so that the end wall 24 together with the inner panel and the gasket define all the required valves and flow channels.

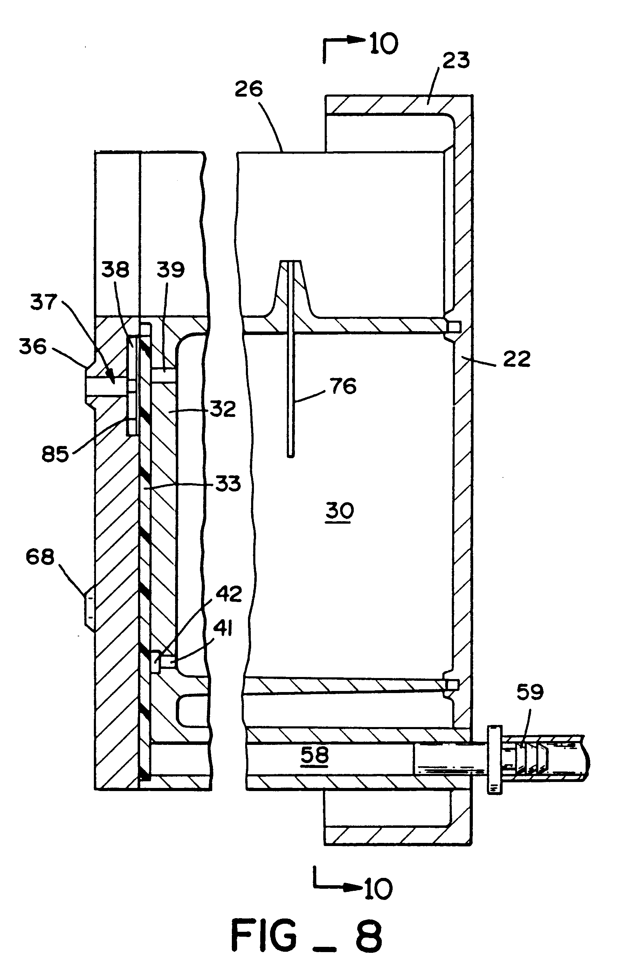

Extending distally from the end wall 24 is a vacuum connector nipple 36, as shown in Figure 8. The nipple 36 is disposed to be engaged by the surgical instrument control machine, and a passage 37 leads therefrom through the end wall 24 to a filter well 38. A hydrophobic filter membrane 85 is received in the well 38, and is clamped at its outer edge between the end wall 24 and the gasket 33. From the filter well 38 a passage 39 in the inner panel 32 leads directly to the sealed volume of the small container 30. The connector 36 receives the controlled, variable vacuum from the surgical machine, and transfers this vacuum directly to the small container 30.

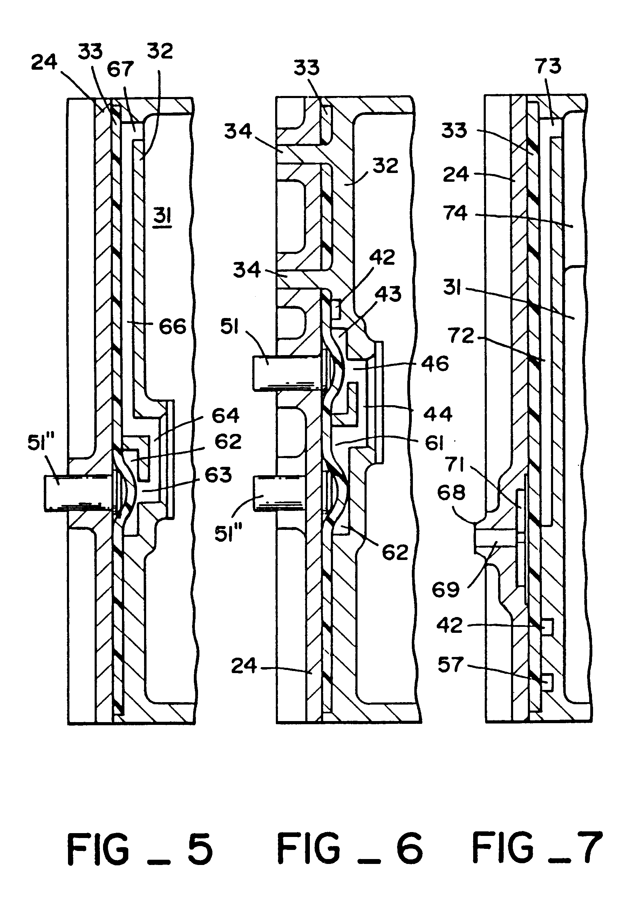

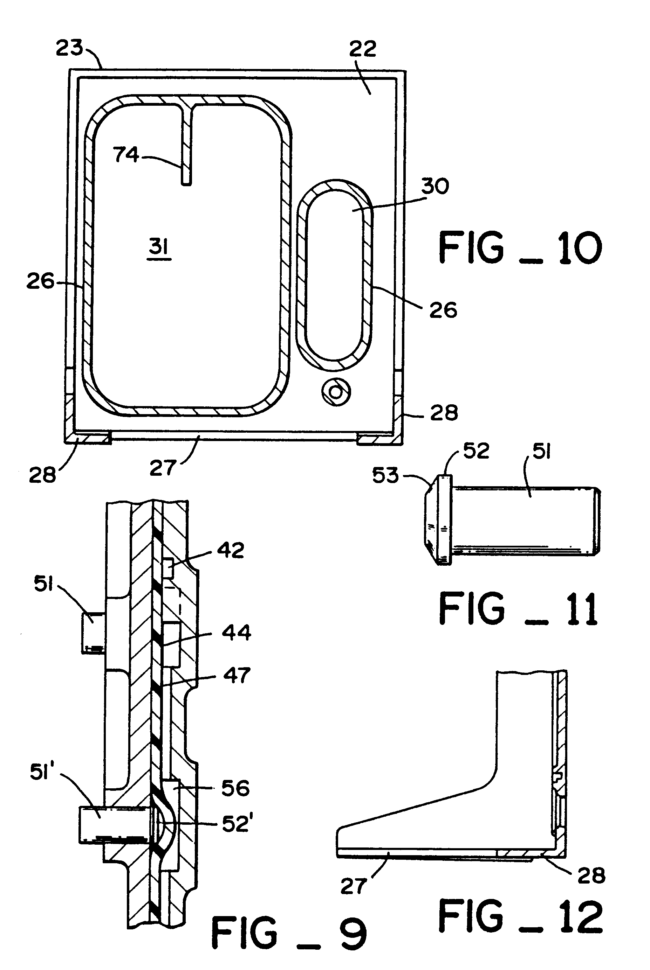

A port 41 in the lower end of the container 30 extends to a flow channel 42 formed in the panel 32, as shown in Figures 4 and 8. The flow channel 42 extends obliquely to the vicinity of a valve chamber 43, which comprises a cylindrical cavity formed in the inner panel 32. The channel 42 extends about the periphery of the valve chamber 43 for more than half the circumference thereof, and joins a recessed flow passage 44, as also shown in Figure 6. The passage 44 leads to a port 46 extending axially into the valve chamber 43. A flow channel 47 extends in the inner panel 32 to join the valve chamber 43 and the cylindrical chamber 56 in a tangential relationship, as also shown in Figures 4 and 9.

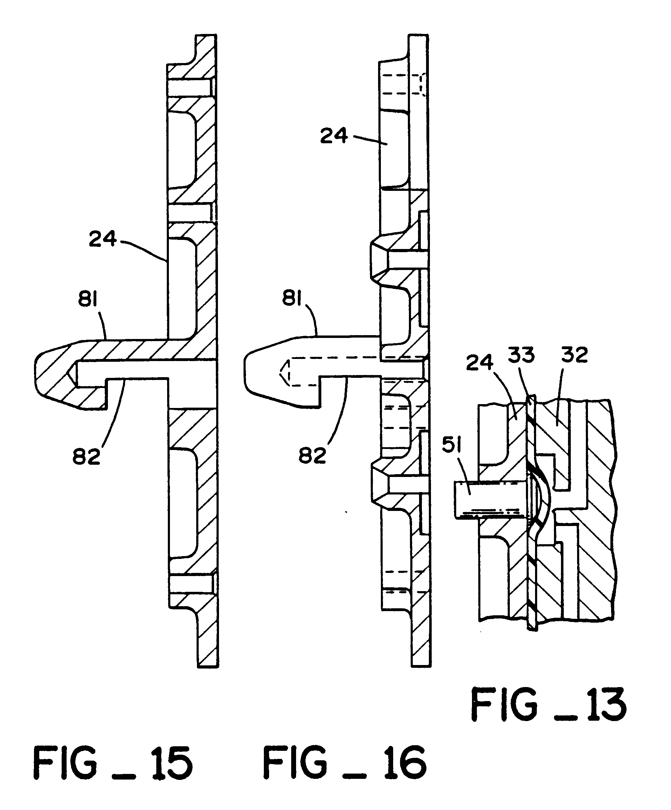

A valve stem actuator 51 extends outwardly through a hole in the end wall 24, and is joined at the inner end of a piston head 52, as also shown in Figures 6 and 11. The inner end of the piston head has a chamfered portion 53 to present a smoothly contoured surface to inpinge upon the gasket 33. The head 52 is smaller in diameter than the valve chamber, and the stem 51 is aligned axially therewith. It may be appreciated that inward translation of the stem 51 drives the head 52 to deform the gasket 33 elastically, urging the gasket material to block the port 46 and prevent fluid flow through the valve chamber. See also Figure 13. The elastic nature of the gasket material opposes the inward translation of the valve stem, and assures that the valve will be biased to the normally open position to restore fluid flow when the valve stem is released. The valve stem is actuated by a mechanism of the surgical instrument operating machine, which forms no part of the present invention.

The flow channel 47 leads to a cylindrical chamber 56 similar in configuration to the valve chamber 43. Another flow channel 57 extends from the chamber 56, and leads to a delivery tube 58 which extends to the outer end wall 22 (Figure 8). A tubing nipple 59 may be mounted on the outer end of the delivery tube 58 to connect to the aspiration tube of a surgical instrument or probe. The flow channels 47 and 57 enter the chamber 56 in diametrically opposed relationship.

A valve stem 51 extends through the end wall 24, and functions as its counterpart 51 to deform the gasket portion upon which it impinges and urge that portion into the chamber 56, as shown in Figure 9. However, the gasket portion in the chamber 56 does not block the fluid flow therethrough, due to the diametrical relationship of the flow channels entering therein. Rather, the piston 52 is provided to create a back pressure wave, or reflux, whenever the vacuum delivered to the surgical instrument is shut off by the valve 43.

To summarize the function of the components described thus far, the controlled variable vacuum provided to the small container 30 by the connector 36 is delivered through the channel 42, valve 43, chamber 56, channel 57, and delivery tube 58 to the aspirating surgical instrument. When the surgeon desires to cease the cutting action of the instrument, a hand or foot control is actuated to activate the valve stem 51 and close the valve 43. Thus any further vacuum induction of tissue into the surgical instrument is stopped abruptly. However, a vacuum condition may remain in the fluid channel extending from the valve 43 to the surgical instrument. This residual vacuum may act to retain tissue in the cutting instrument; when the surgeon withdraws the instrument from the surgical site, the tissue may be torn or damaged. This effect can be especially serious in delicate eye surgery and the like.

Thus, the reflux action of the chamber 56 is an important feature of the present invention in preventing such accidental damage. Whenever the valve 43 is closed to stop the vacuum aspiration, the valve stem 51 is actuated to drive the gasket 33 into the chamber 56, thereby displacing a small amount of the fluid in the chamber into the fluid flow path from the surgical instrument to the valve 43. The result is that the residual vacuum is alleviated, and any tissue retained in the cutting instrument is released.

The distal end of the arcuate portion of the flow channel 43 is also connected through a short channel portion 61 to a valve chamber 62. The valve chamber 62 is configured similarly to the chamber 43, and a valve stem actuator 51 is translatably secured in the end wall 24 and disposed to function like its counterpart 51 described previously. The axially disposed port 63 of the chamber 62 connects to a recessed flow channel 64, and thence to a flow channel 66, as shown in Figure 4 and 5. The flow channel 66 terminates in a port 67 which opens into the large container 31. Thus a fluid flow path from the small container to the large container is defined through channels 42 and 61, valve chamber 62, channels 64 and 66, and port 67, with the valve 62 being interposed to selectively block the flow path.

Also extending outwardly from the end wall 24 is another vacuum connector nipple 68. Like the connector 36, the connector 68 is positioned to be connected with a port in the surgical machine, and is provided with a generally constant vacuum by the machine. The connector 68 is joined by a passage 69 to a filter well 71 which is also covered by a hydrophobic membrane 85, and thence through a flow channel 72 to a port 73 in the large container 31 (Figures 4 and 7). Thus the constant vacuum applied to the connector 68 is delivered directly to the large container 31. It should be noted that the container 31 includes a septum wall 74 which separates the vacuum port 73 and the fluid intake port 67, so that no fluid inadvertently enters the port 73. Also, the hydrophobic membrane permits gas flow into the surgical machine, but prevents aspiration of any liquid into the surgical machine.

To summarize the operation of the cassette of the first preferred embodiment of the present invention, the controlled, varible vacuum level of the surgical machine is applied to the connector 36 and conducted directly to the small volume of the container 30. To provide vacuum aspiration to a surgical instrument connected to the nipple 59, the valve 43 is open to permit vacuum connection from the small container through flow channel 42, valve 43, channels 47 and 57 to the delivery tube connected to the instrument. At the same time the valve 62 is closed, thus isolating the large and small containers.

Whenever the surgeon desires to curtail the vacuum aspiration effect, even momentarily, the surgeon's controller activates the machine to close valve 43, thus interrupting the aspiration flow path to the surgical instrument. At the same time, the valve stem 51 is actuated to displace fluid in the aspiration channel and alleviate the residual vacuum therein to release any tissue entrained in the surgical cutting instrument. Also at the same time, the valve 62 is opened, thus connecting the two containers through the channels 42 and 47, the valve 62, and the channel 66. The vacuum level constantly applied to the connector 68 causes any fluid above the port 41 of the small container to be drawn rapidly into the large container through the open flow channel just described, thus emptying the small container.

The small container also includes a pair of electrodes 76 which extend into the upper portion of the small container and are connected to a circuit in the surgical machine which senses fluid immersing the two electrodes. Whenever fluid bridges the two electrodes 76, the machine temporarily disables the surgical instrument, closes valve 43, actuates the reflux device 56, and opens the valve 62 to drain the small container. This process requires a very brief time to complete, and assures that the small container will not fill to the level of the port 39, thus preventing fluid aspiration into the surgical machine.

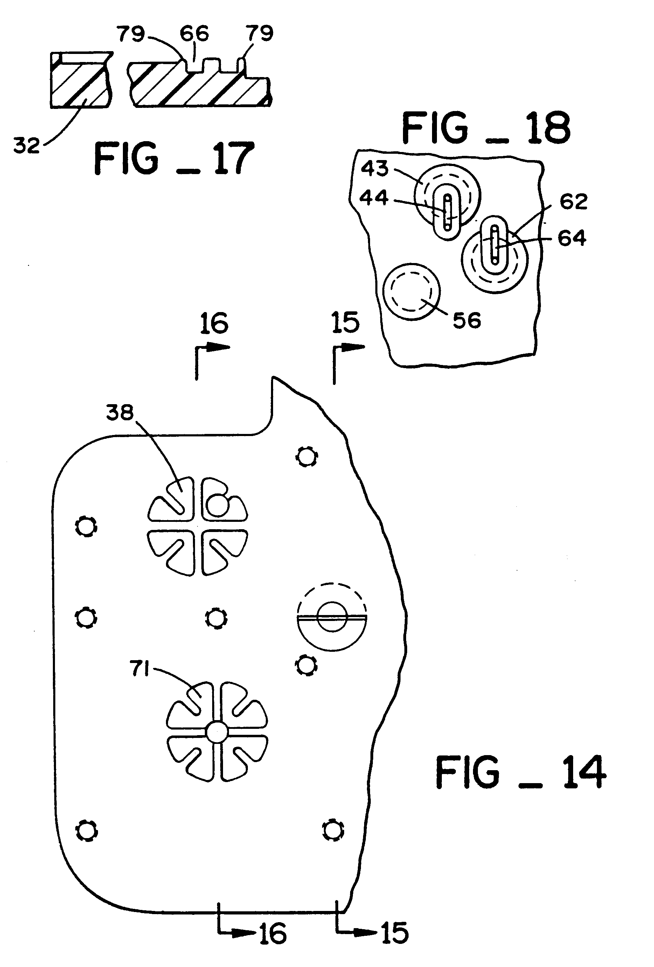

With regard to Figure 17, it should be noted that all of the flow channel and valve chamber features described herein include a raised lip 79 extending continuously from the inner panel 32 and impinging on the gasket 33. The lip assures a vacuum-tight seal with the gasket, and is integrally molded with the panel 32.

The cassette 21 also includes a pilot pin 81 extending outwardly from the end wall 24, as shown in Figures 1-3 and 15-16. Th pilot pin is disposed to be received by a complementary part of the surgical machine, and is provided to guide and secure the engagement of the connectors 36 and 68 with the machine. The pin 81 includes a detent slot 82 spaced proximally from the end thereof and adapted to be engaged by a latch mechanism of the surgical machine to secure the cassette to the machine in the operative position.

The cassette of the present invention is easily formed by plastic molding techniques, and may be manufactured inexpensively. Thus the cassette is suitable for single usage, and disposal thereafter. A plugged port 84 extending through outer wall 22 to the large container 31 is provided so that the aspirant fluid and tissue debris may be withdrawn for pathology analysis and the like.

Although the tubeless vacuum aspiration collection cassette of the present invention has been described above in connection with a preferred embodiment in which a pair of containers is utilized, it will be appreciated by those skilled in the art that the flow passage means, valve means, valve actuator means, and other important features of the present invention are adaptable for use in cassettes which utilize either a single container or more than two containers.

It is also to be appreciated by those skilled in the art that the flow passage means, valve means, valve actuator means, and other important features of the present invention are adaptable for use in cassettes for the selective control of fluids, in addition to air under vacuum, which are used in surgical procedures. Such examples include, but are not limited to, infusion fluid and pressurized air.

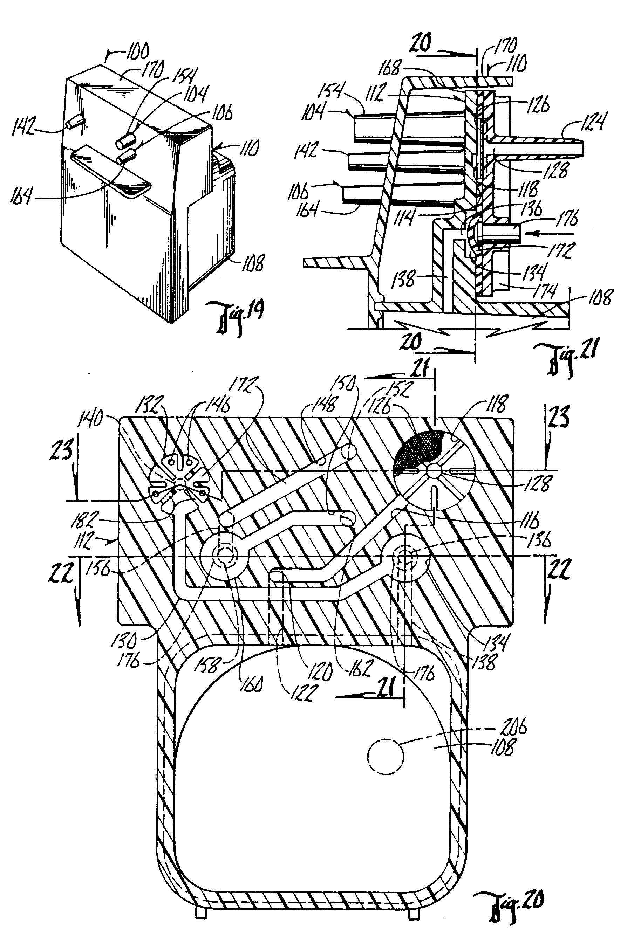

A second preferred embodiment of the present invention is therefore shown in Figures 19-24, and described as follows.

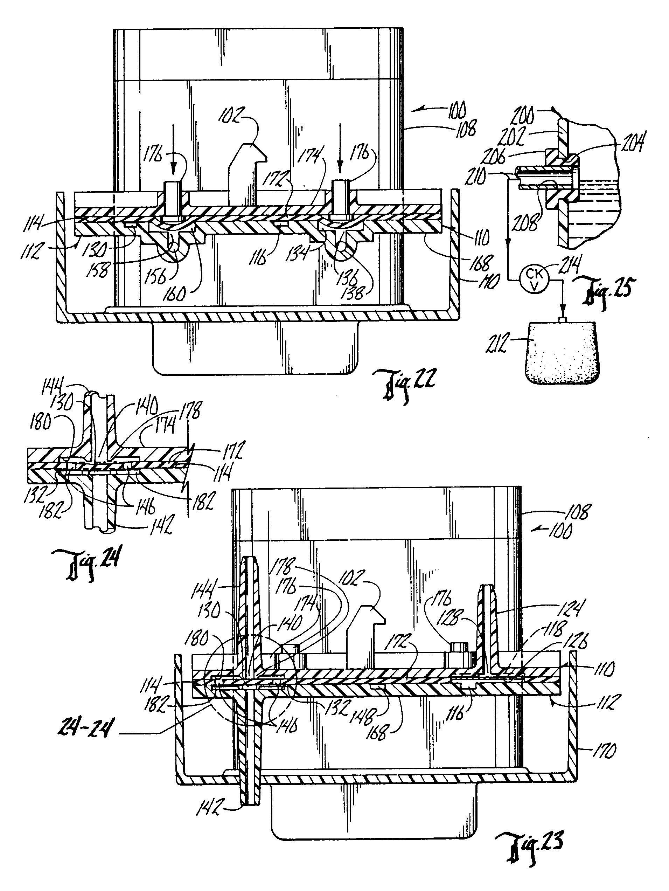

With particular reference to Figures 19-21, the second preferred embodiment of the present invention comprises a cassette 100 substantially similar in design and operation to cassette 21 of Figures 1-18. A pilot pin 102 (see Figures 22 and 23), like pilot pin 81 of the first preferred embodiment, locks cassette 100 to the surgical machine.

Cassette 100, like cassette 21, can be connected to tubing conduit extending to a surgical aspiration tool, and is also connectable to an aspiration source in the surgical machine. Flow passage means, valve means, and valve actuator means, substantially the same as with regard to the first preferred embodiment, allow control of direction of aspiration to the aspiration tool the control and direction of aspiration fluid and depris to a collection container 108 disposed within cassette 100.

Cassette 100 also includes the additional optional feature of allowing selective control of infusion fluid between an infusion fluid source and conduit tubing to an infusion fluid surgical tool. Cassette 100 includes an infusion fluid inlet 104 and an infusion fluid outlet 106 connectible to an infusion fluid source and tool respectively. Flow passage means, valve means, valve actuator means, substantially similar to those shown in and described with regard to the first preferred embodiment of the present invention, are utilized to control infusion fluid to the infusion fluid surgical tool.

It is to be understood that the elements and operation of cassette 100 function substantially in the same way as described with regard to the first preferred embodiment of the present invention, and therefore reference should be had to that description and will not be repeated herein.

The primary differences between cassette 100 and cassette 21 are summarized as follows. As shown in Figure 20, a single fluid collection container 108 is disposed within cassette housing 110. A first wall portion 112 of cassette housing 110 extends above container 108. As is most clearly shown in Figure 20, a plurality of fluid flow channels are formed in the machine-facing surface 114 of wall portion 112. A first flow channel 116 connects circular filter well 118 to a port 120. Port 120 is in turn in communication with bore 122 which is in turn is in communication with the interior of container 108. As shown in Figure 21, a connecting nipple 124 is in fluid communication with circular filter well 118 containing a hydrophobic filter membrane 126. Nipple 124 automatically connects to the aspiration source of the machine when cassette 100 is connected and secured to the machine. First flow channel 116, port 120, and bore 122, in conjunction with filter well 118 and port 128 associated with connecting nipple 124 therefore supply aspirating vacuum to container 108.

A second flow channel 130 in first wall portion 112 extends between segmented well 132 and valve chamber 134 which surrounds port 136. Port 136 in turn is in fluid communication with bore 138 which fluidly communicates with container 108. Segmented well 132 is in fluid communication with port 140 which is associated with aspiration tool nipple 142. The fluid pathway through segmented well 132, second flow channel 130, and bore 138 provides a pathway for fluid and debris aspirated from the surgical aspiration tool into the collection container 108. Valve chamber 134 cooperates with gasket member 172 and valve stem 176, in the same way as previously described with regard to the first preferred embodiment, to allow closing and opening of this fluid pathway.

A third flow channel 148 and fourth flow channel 150 are formed in first wall portion 112. Third flow channel 148 at one end communicates with port 152 which in turn communicates with infusion fluid input nipple 154. The other end of third flow channel 148 communicates with recessed flow channel 156 which in turn communicates with port 158 in the center of valve chamber 160. Fourth flow channel 150 communicates at one end with port 162 which in turn communicates with infusion fluid outlet nipple 164. The other end of fourth flow channel 150 communicates directly with valve chamber 160. Valve chamber 160, like valve chamber 134 and those described with regard to the first preferred embodiment of the invention cooperate with gasket 172 and valve stem 176 to selectively block port 158 and stop fluid flow between third and fourth flow channels 148 and 150. Thus, the surgical machine can also control infusion fluid to an infusion fluid tool.

Aspiration tool nipple 142, infusion fluid input nipple 154, and infusion fluid outlet nipple 164 extend from the surface 168 of wall portion 112 which faces away from the surgical machine. They extend through appropriate aperatures in a manifold hood 170 which extends up and overfirst wall portion 112. Hoses or tubing conduit, as appropriate are connected to the nipples as desired. Figures 21-23 show how gasket membrane 172 is sandwiched over the flow channels wells, and valve chambers in first wall portion 112 by a backplate 174. Backplate 174 can be secured to first wall portion 112 similarly to that described with regard to the first preferred embodiment of the invention.

It is also to be understood that the second preferred embodiment includes a vent and check valve to vent the aspiration line to the surgical aspiration tool to atmosphere. By referring to Figures 20, 23, and 24, the check valve and vent can be described. A vent nipple 144 surrounds a port 140 and extends outwardly from back plate 174. Vent nipple 144 connects with a venting valve in the machine when cassette 100 is secured in the machine. The venting valve is connected to atmosphere, is controlled by the machine, and operates as is known in the art.

Port 140 has a raised lip 178 surrounding it, which in turn is surrounded by a well 180. As can be seen in Figures 23 and 24, port 140, lip 178, and well 180 align with segmented well 132 in first wall portion 112, but are separated from it by gasket 172. Although small apertures 146 exist in gasket 172, fluid and debris traveling through aspiration nipple 142 into second flow channel 130, and on to container 108, can not enter vent nipple 144 because when the venting valve in the machine is closed, gasket 172, in its normal state, covers and seals over raised lip 178 around port 140. The positioning of the four small apertures 146 can be seen in Figure 20. It is also pointed out that spoke members 182, which extend from first wall portion 112 into segmented well 132, keep gasket 172 from blocking the flow path through aspiration 142.

Figure 24 shows that when aspiration is stopped to the aspiration tool, and the venting valve in the machine is opened, the atmospheric pressure causes gasket 172 to move away from lip 178 and the aspiration line is vented to atmosphere through small apertures 146. This assists in diminishing residual vacuum in the aspiration line.

Figure 25 shows an additional modification which can be made to the cassettes of the invention to provide auxilliary storge of fluid or debris aspirated into the collection container, such as container 31 of the first described preferred embodiment, or container 108 of the second described preferred embodiment. Figure 25 shows a cassette 200 in accordance with the present invention with a fluid container 202 disposed therein. An aperture 204 is in fluid communication with the interior of container 202. It is preferred that aperture 204 be at least 1/2" below the top of container 202. A plug 206 is sealingly positioned in aperture 204, but also has an aperture 208 into which a tube 210 is sealingly mounted. The opposite end of tube 210 is connected in fluid communication with sealed auxillary bag 212. A check valve 214 is interposed along tube 210. Check valve 214 allows fluid and debris to drain from container 202 to bag 212 when the level of fluid and debris in container 202 reaches aperture 208 in plug 206. Such drainage is by gravity. Check valve 214 disallows, however, passage of fluid, debris, or air from bag 212 back to container 202 when container 202 is under vacuum.

Such check valves are well-known to those of ordinary skill in the art and can take on various configurations while accomplishing the objects above described. An example is a diaphram check valve which allows fluid flow in one direction, but disallows it in another. A further example is a duckbill check valve. Other types could be used as is known in the art.

The auxiliary storage system described above is particularly useful in phaco-emulsification ophthalmic procedures, but is applicable to most, if not all, ophthalmic procedures.

It will be appreciated that the present invention can take many forms and embodiments. The scope of this invention is defined in the appended claims, and it is not intended that the embodiments of the invention presented herein should limit the scope thereof.

## Claims

1. A fast response vacuum aspiration system for use with a source of aspiration and an aspiration surgical instrument, comprising a removable cassette housing (21 or 110) which includes a wall portion, (32 or 112) having a first surface (32 or 114) which, when in use, is oriented towards the source of aspiration, at least one sealed collection container (30, 31 or 108) secured within the cassette housing (21 or 110), one or more flow channels (47, 57, 42, 44, 116 or 130) formed in the first surface (32 or 114) of the wall portion (32 or 112) of the cassette housing (21 or 110) for communicating the container (30, 31 or 108) with the source of vacuum and the aspiration tool, characterized in that a resilient gasket (33 or 172) abuts the first surface (32 or 114) of the wall portion (32 or 112) and covers the fluid channels (47, 57, 42, 44, 116 or 130), a retaining plate (24 or 174), associated with the cassette housing (21 or 110), sandwiching the gasket (33 or 172) to the wall portion (32 or 112) and one or more valve seats (43, 46, 134 or 136) interposed along the flow channels (47, 57, 42, 44, 116 or 130) adapted to receive a valve actuator means (51, 52 or 176) through the retaining plate (24 or 174) to force a portion of the gasket (33 or 172) into the valve seat (43, 46, 134 or 136) blocking any fluid flow in the flow channels (47, 57, 42, 44, 116 or 130).

2. A system according to claim 1, characterized in that the cassette housing includes a pair of sealed containers (30 and 31) formed in the cassette housing (21).

3. A system according to claim 2, characterized in that one (31) of the pair of containers (30 and 31) is substantially greater in volume than the other (30).

4. A system according to claim 1, characterized in that the gasket (33 or 172) is elastically deformable to resiliently bias the valve actuator means (51, 52 or 176) through a position where fluid flow in the fluid channel (47, 57, 42, 44, 130 or 116) is not blocked.

5. A system according to claim 1, characterized in that reflux pressure generator (51, 52, 33, 56 or 144, 172, 178, 142, 180, 182) is formed in the cassette hosing (21 or 110) adapted to generate a positive pressure pulse to at least a portion of a flow channel (47, 57, 42, 44, 130 or 116).

6. A system according to claim 5, characterized in that the reflex pressure generator (51 prime, 52 prime, 33, 56) includes a sealed chamber (56) formed in the cassette housing (21), an inlet and outlet (57 and 47) extending towards the sealed chamber (56).

7. A system according to claim 1, characterized in that the wall portion (32 or 112) is disposed to abut the source of aspiration and a latching means (81) is included for selectively joining the wall portion (32 or 112) to the aspiration source.

8. A system according to claim 2, characterized in that it includes means (68, 69, 71, 72, 73) for connecting one of the containers (31) to a constant vacuum source.

9. A system according to claim 2, characterized in that it includes means for connecting one of the containers (30) to a controlled and variable vacuum source.

## Patentansprüche

1. Schnellansprechendes Vakuum-Aspirationssystem zur Anwendung mit einer Aspirationsquelle und einem Operations-Aspirationsinstrument mit einem entfernbaren Kassettengehäuse (21 oder 110), das einen Wandabschnitt (32 oder 112) mit einer ersten Oberfläche (32 oder 114), die beim Gebrauch zu der Aspirationsquelle gerichtet ist, zumindest einem in dem Kassettengehäuse (21 oder 110) angebrachten abgedichteten Sammelbehälter (30,31 oder 108), einem oder mehreren in der ersten Oberfläche (32 oder 114) des Wandabschnittes (32 oder 112) des Kassettengehäuses (21 oder 110) ausgebildeten Kanälen (47,57,42,44,116 oder 130) zum Verbinden des Behälters (30,31 oder 108) mit der Vakuumquelle und dem Aspirationswerkzeug,
dadurch gekennzeichnet,
daß eine federnde Dichtung (33 oder 172) an der ersten Oberfläche (32 oder 114) des Wandabschnitts (32 oder 112) anliegt und die Fluidkanäle (47,57,42,44,116 oder 130) abdeckt, eine dem Kassettengehäuse (21 oder 110) zugeordnete Halteplatte (24 oder 174), mit der die Dichtung (33 oder 172) schichtartig auf den Wandabschnitt (32 oder 112) und einem oder mehreren Ventilsitzen (43,46,134 oder 136) aufgebracht ist, die entlang der Strömungskanäle (47,57,42,44,116 oder 130) angeordnet sind und die so ausgebildet sind, daß sie eine Ventilbetätigungsvorrichtung (51,52 oder 176) durch die Halteplatte (24 oder 174) aufnehmen, wodurch ein Abschnitt der Dichtung (33 oder 172) in den Ventilsitz (43,46,134 oder 136) gedrückt ist und jeglicher Fluidstrom in den Strömungskanälen (47,57,42,44,116 oder 130) gesperrt ist.

2. System nach Anspruch 1,
dadurch gekennzeichnet,
daß das Kassettengehäuse ein Paar in dem Kassettengehäuse (21) ausgebildete abgedichtete Behälter (30 und 31) aufweist.

3. System nach Anspruch 2,
dadurch gekennzeichnet,
daß einer (31) des Paares der Behälter (30 und 31) wesentlich größeres Volumen als der andere hat.

4. System nach Anspruch 1,
dadurch gekennzeichnet,
daß die Dichtung (33 oder 172) zum federnden Vorspannen der Ventilbetätigungsvorrichtung (51,52 oder 176) in einer Position, in der der Fluidstrom in den Fluidkanal (47,57,42,44,130 oder 116) nicht gesperrt ist, elastisch verformbar ist.

5. System nach Anspruch 1,
dadurch gekennzeichnet,
daß eine Rückflußdruck-Erzeuger (51,52,33,56 oder 144, 172,178,180,182) in dem Kassettengehäuse (21 oder 110) gebildet ist, der so ausgelegt ist, daß er zumindest in einem Abschnitt eines Strömungskanals (47,57,42,44,130 oder 116) einen positiven Druckpuls erzeugt.

6. System nach Anspruch 5,
dadurch gekennzeichnet,
daß der Reflex-Druckerzeuger (51',52',33,56) eine in dem Kassettengehäuse (21) ausgebildeten abgedichtete Kammer (56) aufweist sowie einen sich zur abgedichteten Kammer (56) erstreckenden Einlaß und Auslaß (57 und 47) aufweist.

7. System nach Anspruch 1,
dadurch gekennzeichnet,
daß der Wandabschnitt (32 oder 112) derart angeordnet ist, daß er an die Aspirationsquelle angeordnet und daß ein Schnappelement (81) zum selektiven Verbinden des Wandabschnittes (32 oder 112) mit der Aspirationsquelle vorhanden ist.

8. System nach Anspruch 1,
dadurch gekennzeichnet,
daß es eine Vorrichtung (68,69,71,72,73) zum Verbinden eines der Behälter (31) mit einer kosntanten Vakuumquelle aufweist.

9. System nach Anspruch 2,
dadurch gekennzeichnet,
daß es eine Vorrichtung zum Verbinden eines der Behälter (30) mit einer gesteuerten und variablen Vakuumquelle aufweist.

## Revendications

1. Un système d'aspiration à réponse rapide pour utilisation avec une source d'aspiration et un instrument chirurgical d'aspiration, comprenant un boîtier de cassette amovible (21 ou 110) qui comporte une partie en cloison (32 ou 112) ayant une première surface (32 ou 114) qui, lorsqu'elle est en utilisation, est orientée vers la source d'aspiration, au moins un conteneur collecteur scellé (30, 31 ou 108) fixé dans le boîtier de cassette (21 ou 110), un ou plusieurs canaux d'écoulement (47, 57, 42, 44, 116 ou 130) formés dans la première surface (32 ou 114) de la partie en cloison (32 ou 112) du boîtier de cassette (21 ou 110) pour mettre en communication le conteneur (30, 31 ou 108) avec la source de vide et l'outil d'aspiration, caractérisé en ce qu'un joint résilient (33 ou 172) aboutit à la première surface (32 ou 114) de la partie en cloison (32 ou 112) et recouvre les canaux de fluide (47, 57, 42, 44, 116 ou 130), une plaque de maintien (24 ou 174), associée avec le boîtier de cassette (21 ou 110), prenant en sandwich le joint (33 ou 172) avec la partie en cloison (32 ou 112) et un ou plusieurs sièges de vanne (43, 46, 134 ou 136) interposés le long des canaux d'écoulement (47, 57, 42, 44, 116 ou 130) conçus pour recevoir un moyen actionneur de vanne (51, 52 ou 176) à travers la plaque de maintien (24 ou 174), pour forcer une partie du joint (33 ou 172) dans le siège de vanne (43, 46, 134 ou 136) interrompant tout écoulement de fluide dans les canaux d'écoulement (47, 57, 42, 44, 116 ou 130).

2. Système selon la revendication 1, caractérisé en ce que le boîtier de cassette comprend une paire de conteneurs scellés (30 et 31), formée dans le boîtier de cassette (21).

3. Système selon la revendication 2, caractérisé en ce qu'un conteneur (31) de la paire de conteneurs (30 et 31) a un volume sensiblement plus grand que l'autre conteneur (30).

4. Système selon la revendication 1, caractérisé en ce que le joint (33 ou 172) est déformable élastiquement pour décentrer de façon résiliente les moyens actionneurs de vanne (51, 52 ou 176) jusqu'à une position où l'écoulement du fluide dans le canal de fluide (47, 57, 42, 44, 130 ou 116) n'est pas interrompu.

5. Système selon la revendication 1, caractérisé en ce qu'un générateur de pression de reflux (51, 52, 33, 56 ou 144, 172, 178, 142, 180, 182) est formé dans le boîtier de cassette (21 ou 110) conçu pour générer une impulsion de pression positive à au moins une partie d'un canal d'écoulement (47, 57, 42, 44, 130 ou 116).

6. Système selon la revendication 5, caractérisé en ce que le générateur de pression de reflux (51', 52', 33, 56) comprend une enceinte scellée (56) formée dans le boîtier de cassette (21), une admission et un échappement (57 et 47) s'étendant vers l'enceinte scellée (56).

7. Système selon la revendication 1, caractérisé en ce que la partie en cloison (32 ou 112) est positionnée pour aboutir à la source d'aspiration et un moyen de verrouillage (81) est inclu pour joindre sélectivement la partie en cloison (32 ou 112) à la source d'aspiration.

8. Système selon la revendication 2, caractérisé en ce qu'il comprend des moyens (68, 69, 71, 72, 73) pour connecter l'un des conteneurs (31) à une source de vide constante.

9. Système selon la revendication 2, caractérisé en ce qu'il comprend des moyens pour connecter l'un des conteneurs (30) à une source de vide commandée et variable.
